# EUROPÄISCHE PATENTSCHRIFT

(11) **EP 1 946 718 B1**
(45) Veröffentlichungstag und Bekanntmachung des Hinweises auf die Patenterteilung: **09.06.2010**
(21) Anmeldenummer: 07001209.1
(22) Anmeldetag: 19.01.2007
(51) Int. Cl.: A61B 19/00, A61B 5/103

(54) **Registrierung und Stabilitätstest eines Knies durch Aufnahme zweier Punkte an dem Knie**
Registration and stability test of a knee by recording two points on the knee
Enregistrement et test de stabilité d'un genou par l'enregistrement de deux points sur le genou

(43) Veröffentlichungstag der Anmeldung: 23.07.2008
(73) Patentinhaber: BrainLAB AG, 85622 Feldkirchen (DE)
(72) Erfinder: Warkentine, Blaine, Long Beach, CA 90803 (US); Huber, Hansjoerg, 80805 München (DE)
(74) Vertreter: Schwabe - Sandmair - Marx

(56) Entgegenhaltungen:
- EP-A- 1 700 574
- DE-A1- 10 313 747
- US-A1- 2004 106 861
- US-A1- 2006 161 052

## Beschreibung

Die vorliegende Erfindung bezieht sich auf ein Verfahren und eine Vorrichtung zum Registrieren eines Knies oder Kniegelenks, insbesondere vor und nach einer Knie-Operation, und zum Testen der Stabilität oder Beweglichkeit des Knies oder Kniegelenks und zur Berechnung der Isometrie des Knies oder Kniegelenks, insbesondere vor und/oder nach einer Knie-Operation, wobei hierfür lediglich zwei Punkte im Bereich des Knies oder Kniegelenks mittels eines Pointers aufgenommen werden müssen, dessen Position und/oder Orientierung mittels eines medizinischen Navigationssystems ermittelt werden kann.

Ein derartiges Verfahren und System wird in EP 1 700 574 offenbart.

Damit ein Operateur während einer Operation eine bessere Übersicht hat, wird eine Knie- oder Kniegelenks- oder Kreuzband-Operation häufig bei einer Blutsperre durchgeführt. Diese Blutsperre sollte jedoch, um gesundheitliche Folgen oder Schäden zu vermeiden, nicht länger als 60-90 Minuten aufrecht erhalten werden. Müssen nun während der Blutsperre z.B. intraoperativ zahlreiche Punkte an dem Knie aufgenommen werden, um das Knie registrieren und bezüglich des Knies computergestützt navigieren zu können oder um Beweglichkeitsinformationen des Knies zu gewinnen, verringert sich die für die Operation zur Verfügung stehende Zeit erheblich.

Es ist demnach eine Aufgabe der vorliegenden Erfindung die Nachteile aus dem Stand der Technik zu überwinden. Es ist weiter eine Aufgabe der vorliegenden Erfindung ein Verfahren und eine Vorrichtung bereitzustellen, welche eine einfache und schnelle Registrierung eines Knies oder Kniegelenks und eine schnelle Ermittlung der Beweglichkeit und Stabilität eines Knies oder Kniegelenks ermöglichen.

Diese Aufgabe wird durch den Gegenstand der unabhängigen Ansprüche gelöst. Vorteilhafte Ausführungsformen ergeben sich aus den abhängigen Ansprüchen.

Bei dem erfindungsgemäßen Verfahren zur Registrierung und zur Ermittlung der Stabilität und/oder Beweglichkeit eines Knies oder Kniegelenks oder Kreuzbandes eines Patienten werden mittels eines navigierten Instruments, wie zum Beispiel mittels eines Pointers, wie eines mittels Berührung oder berührungslos arbeitenden Pointers, oder mittels eines navigierten Bohrers, einer Bohrhülse oder eines Drill Guides, ein erster Referenzpunkt auf der Tibia, insbesondere ein Punkt auf auf der Haut der anterioren Tibia oder auf dem anterior liegenden Teil der Tibia oder auch unmittelbar auf der anterioren Tibia oder auf dem anterior liegenden Teil der Tibia, des Patienten abgetastet oder aufgenommen und mittels des Pointers ein zweiter Referenzpunkt auf der Tibia oder der Patella, insbesondere ein Punkt auf dem inferioren Ende der Patella, oder Kniescheibe des Patienten abgetastet oder aufgenommen, wobei an der Tibia des Patienten ein erster Referenzstern angeordnet ist oder vor dem Abtasten angeordnet wird und an dem Femur des Patienten ein zweiter Referenzstern angeordnet ist oder vor dem Abtasten angeordnet wird. An dem Pointer ist eine Trackingreferenz, wie ein Referenzstern, angeordnet, welche IR-Strahlung reflektierende oder emittierende Marker aufweist. Mit Hilfe der Trackingreferenz kann ein Navigationssystem den Pointer verfolgen, die Position und/oder Orientierung der Trackingreferenz und des Pointers erfassen, und die Position und/oder Orientierung des Pointers im Raum ermitteln. Werden mittels des Pointers die zwei Referenzpunkte aufgenommen, wird mittels des Navigationssystems die Position und/oder Orientierung des Pointers während des Abtast- oder Aufnahmevorganges des ersten und des zweiten Referenzpunktes mit Hilfe der an dem Pointer angeordneten Trackingreferenz von dem Navigationssystem erfasst und ermittelt, so dass dem Navigationssystem die Position und/oder Orientierung des Pointers während des Abtast- oder Aufnahmevorganges des ersten und des zweiten Referenzpunktes bekannt ist. Aus der Position und/oder Orientierung des Pointers während des Abtastvorganges wird die Position des ersten und des zweiten Referenzpunkts bezüglich eines an der Tibia angeordneten ersten Koordinatensystems und/oder bezüglich eines an dem Femur angeordneten zweiten Koordinatensystems ermittelt. Das erste Koordinatensystem kann beispielsweise in oder im Bereich der Kniemitte liegen oder kann zumindest im Bereich des ersten Referenzsterns liegen. Das zweite Koordinatensystem kann in oder im Bereich der Kniemitte liegen oder kann zumindest im Bereich des zweiten Referenzsterns liegen.

Ist die Position des ersten und des zweiten Referenzpunktes dem Navigationssystem bekannt, wird die Position des ersten Referenzpunktes um einen vorgegebenen ersten Betrag in einen ersten Achsenpunkt und die Position des zweiten Referenzpunkts um einen vorgegebenen zweiten Betrag in einen zweiten Achsenpunkt insbesondere computergestützt verschoben oder versetzt. Vorzugsweise wird der erste Referenzpunkt um einen Betrag in den ersten Achsenpunkt verschoben oder versetzt, welcher in einem Bereich zwischen 1cm und 3cm, z.B. bei 1.5cm, liegt und der zweite Referenzpunkt wird vorzugsweise um einen Betrag verschoben oder versetzt, welcher in einem Bereich zwischen 2cm und 5cm, z.B. bei 3cm, liegt. Der erste Referenzpunkt wird in den vorzugsweise innerhalb der Tibia liegenden ersten Achsenpunkt bevorzugt in eine Richtung verschoben, welche parallel zu der Richtung oder Orientierung ist, welche von dem Navigationssystem als Orientierung für den Pointer bei dem Abtasten oder Aufnehmen des ersten Referenzpunktes ermittelt wurde. Vorzugsweise werden die Position des ersten Referenzpunktes durch Abtasten des ersten Referenzpunktes mit dem Pointer und die Position und/oder Orientierung des Pointers während des Abtastens des ersten Referenzpunktes von dem Navigationssystem ermittelt, und die ermittelte Information über die Position des ersten Referenzpunktes wird in dem Navigationssystem zwischengespeichert oder weiterverarbeitet, indem diese Position in die Richtung oder Orientierung des Pointers, z.B. um ungefähr 1,5cm, verschoben wird, welche der Pointer bei der Aufnahme des ersten Referenzpunktes hatte. Der zweite Referenzpunkt wird in den vorzugsweise innerhalb des Kniegelenks liegenden ersten Achsenpunkt bevorzugt in eine Richtung verschoben, welche parallel zu der Richtung oder Orientierung ist, welche von dem Navigationssystem als Orientierung für den Pointer bei dem Abtasten oder Aufnehmen des zweiten Referenzpunktes ermittelt wurde. Vorzugsweise werden die Position des zweiten Referenzpunktes durch Abtasten des zweiten Referenzpunktes mit dem Pointer und die Position und/oder Orientierung des Pointers während des Abtastens des zweiten Referenzpunktes von dem Navigationssystem ermittelt, und die ermittelte Information über die Position des zweiten Referenzpunktes wird in dem Navigationssystem weiterverarbeitet, indem diese Position in die Richtung oder Orientierung des Pointers, z.B. um ungefähr 3cm, verschoben wird, welche der Pointer bei der Aufnahme des zweiten Referenzpunktes hatte. Insbesondere kann auch aus dem ersten und dem zweiten Achsenpunkt die mechanische Tibiaachse ermittelt oder approximiert werden, indem die Tibiaachse z.B. durch Verbindung der beiden Achsenpunkte gebildet wird.

Sind die Positionen der Referenzpunkte und der Achsenpunkte ermittelt worden, werden die Tibia und der Femur zumindest nahezu in einen Winkel von 180 Grad relativ zueinander in eine gestreckte Position gebracht, so dass der Femur zumindest nahezu eine Verlängerung der Tibia bildet. In dieser Position ist eine volle oder vollständige Extension zwischen Tibia und Femur erreicht. Vorzugsweise wird in dieser Position der Femur relativ zu der Tibia neutral ausgerichtet, indem z.B. eine bestehende Rotation zwischen Tibia und Femur ausgeglichen wird, so dass die Tibia relativ zu dem Femur unverdreht liegt. Bevorzugt werden mittels des Navigationssystems die Positionen der beiden Referenzsterne und damit die Positionen der Referenz- und Achsenpunkte insbesondere relativ zueinander ermittelt, so dass dem Navigationssystem der Abstand oder die relative Länge zwischen den Referenzpunkten und zwischen den Achsenpunkten bekannt ist. In der gestreckten Position, in welcher vorzugsweise die Tibia und der Femur rotationsneutral relativ zueinander ausgerichtet sind, wird ein Beweglichkeitstest zur Ermittlung der Beweglichkeit der Tibia relativ zu dem Femur durchgeführt. Während des Beweglichkeitstests werden mittels des Navigationssystems durch Ermittlung der Positionen und/oder Orientierungen des ersten und des zweiten Referenzsterns die Positionen des ersten und des zweiten Achsenpunktes relativ zueinander bestimmt. Bei dem Beweglichkeitstest werden bevorzugt ein Rotationsbeweglichkeitstest, ein Translationsbeweglichkeitstest und ein Flexionsbeweglichkeitstest der Tibia und des Femurs relativ zueinander durchgeführt. Bei dem Rotationsbeweglichkeitstest wird die Tibia vorzugsweise relativ zu dem Femur um die Längsachse gedreht oder rotiert und durch Ermittlung der Positionen des ersten Achsenpunkts relativ zu dem zweiten Achsenpunkt wird ermittelt, um welchen Rotationswinkel die Tibia relativ zu dem Femur insbesondere maximal gedreht werden kann. Bei dem Translationsbeweglichkeitst wird die Tibia bevorzugt relativ zu dem Femur senkrecht zu der Längsachse anterior verschoben oder bewegt und durch Ermittlung der Positionen des ersten Achsenpunkts relativ zu dem zweiten Achsenpunkt wird ermittelt, um welchen Translationsbetrag die Tibia relativ zu dem Femur insbesondere maximal verschoben werden kann. Bei dem Flexionsbeweglichkeitstest wird die Tibia relativ zu dem Femur abgewinkelt oder gebeugt und durch Ermittlung der Positionen des ersten Achsenpunkts relativ zu dem zweiten Achsenpunkt wird ermittelt, um welchen Flexionswinkel die Tibia relativ zu dem Femur insbesondere maximal abgewinkelt oder gebeugt werden kann.

Nach dem Beweglichkeitstest, welcher bei voller Extension der Tibia und des Femurs relativ zueinander durchgeführt wurde, werden die Tibia und der Femur um einen vorgegebenen ersten Winkelwert, insbesondere um 30 Grad, relativ zu der gestreckten Position oder der vollen Extension abgewinkelt. In dieser zweiten Position wird wieder der Beweglichkeitstest durchgeführt und mittels des Navigationssystems werden während des Beweglichkeitstests durch Ermittlung der Positionen und/oder Orientierungen des ersten und des zweiten Referenzsterns die Positionen des ersten und des zweiten Achsenpunktes relativ zueinander ermittelt. Vor diesem Beweglichkeitstest werden vorzugsweise die Tibia und der Femur in eine neutrale Rotationsposition relativ zueinander gebracht. Auch dieser Beweglichkeitstest umfasst vorzugsweise den Rotationsbeweglichkeitstest, den Translationsbeweglichkeitstest und den Flexionsbeweglichkeitstest.

Nach dem zweiten Beweglichkeitstest werden die Tibia und der Femur um einen vorgegebenen zweiten Winkelwert, insbesondere um 90 Grad, relativ zu der gestreckten Position abgewinkelt oder um die Differenz zwischen dem zweiten und dem ersten Winkelwert von der vorhergehenden Position abgewinkelt. In dieser Position wird wieder der Beweglichkeitstest durchgeführt und mittels des Navigationssystems werden während des Beweglichkeitstests durch Ermittlung der Positionen und/oder Orientierungen des ersten und des zweiten Referenzsterns die Positionen des ersten und des zweiten Achsenpunktes ermittelt. Auch vor diesem Beweglichkeitstest werden vorzugsweise die Tibia und der Femur in eine neutrale Rotationsposition relativ zueinander gebracht. Die in den verschiedenen Positionen durchgeführten Beweglichkeitstests können auf die gleiche Weise ablaufen oder können sich voneinander unterscheiden. Insbesondere können bei allen Beweglichkeitstests sowohl der Rotationsbeweglichkeitstest, der Translationsbeweglichkeitstest als auch der Flexionsbeweglichkeitstest durchgeführt werden oder es können auch nur einzelne dieser Tests ausgewählt und durchgeführt werden.

Aus den Beweglichkeitstests, welche bei verschiedenen Positionen des Femurs und der Tibia relativ zueinander durchgeführt wurden, insbesondere aus den Positionen der Achsenpunkte, insbesondere relativ zueinander, welche während des Beweglichkeitstests ermittelt wurden, werden die Stabilität und Beweglichkeit des Knies oder Kniegelenks oder des Kreuzbandes ermittelt. Insbesondere werden vor den Beweglichkeitstests die Tibia und der Femur in eine neutrale Position, vorzugsweise in eine neutrale rotatorische und translatorische Position, relativ zueinander gebracht, und die Position der Achsenpunkte relativ zueinander wird in der neutralen Position durch Ermittlung der Position und/oder Orientierung der Referenzsterne in dem Navigationssystem ermittelt. Vorzugsweise wird bei den Beweglichkeitstests die maximale Beweglichkeit des Knies oder Kniegelenks in der jeweiligen Position ermittelt. Ist jeweils die maximale Auslenkung, wie die maximale Rotation oder die maximale Translation oder die maximale Flexion, der Tibia relativ zu dem Femur erreicht, wird die Position der Achsenpunkte relativ zueinander bei der maximalen Auslenkung durch Bestimmung der Position und/oder Orientierung der Referenzsterne von dem Navigationssystem ermittelt. Vorzugsweise werden bei dem Beweglichkeitstest in der vollen Extenstion der Tibia und des Femurs relativ zueinander die Positionen der Achsenpunkte in der neutralen Start- oder Ausgangsposition und in der maximal möglichen Endposition ermittelt. Ebenso werden bevorzugt in der von der vollen Extension um den ersten Winkelwert, z.B. um 30 Grad, und um den zweiten Winkelwert, z.B. um 90 Grad, abgewinkelten Position, die Positionen der Achsenpunkte in der neutralen Start- oder Ausgangsposition und in der maximal möglichen Endposition ermittelt. Aus diesen ermittelten Punkten wird von dem Navigationssystem bevorzugt die Stabilität oder Beweglichkeit des Knies oder Kniegelenks bestimmt.

Auch können in der gestreckten Position der Tibia und des Femurs relativ zueinander, in der um 30 Grad von der gestreckten Position abgewinkelten Position und in der um 90 Grad von der gestreckten Position abgewinkelten Position mittels des Navigationssystems durch Ermittlung der Positionen und/oder Orientierungen des ersten und des zweiten Referenzsterns die Positionen und/oder Orientierungen der aus dem ersten und zweiten Achsenpunkt ermittelten oder approximierten Tibiaachse vorzugsweise in der Start- und Endposition während der Beweglichkeitstests ermittelt werden. Aus den Positionen und/oder Orientierungen der approximierten Tibiaachse kann die Knie- oder Kniegelenkbeweglichkeit ermittelt werden. Auch kann aus der Tibiaachse durch Transformation oder Spiegelung der Tibiaachse, insbesondere an dem zweiten Achsenpunkt, z.B. dem Kniemittelpunkt, die Femurachse approximiert werden, deren Position und/Orientierung auch vorzugsweise in der Start- und Endposition verfolgt und ermittelt werden kann.

Vorzugsweise wird als eine Achse des ersten Koordinatensystems, bezüglich dessen die Referenzpunkte und Achsenpunkte definiert oder registriert werden können, die Tibiachse verwendet und als eine weitere Achse des ersten Koordinatensystems wird vorzugsweise eine Achse verwendet, welche parallel zu der Orientierung des Pointers während der Aufnahme des ersten Referenzpunktes verläuft. Als eine Achse des zweiten Koordinatensystems, bezüglich dessen die Referenzpunkte und Achsenpunkte definiert oder registriert werden können, wird bevorzugt die Femurachse verwendet und als eine weitere Achse des zweiten Koordinatensystems wird bevorzugt eine Achse verwendet, welche parallel zu der Orientierung des Pointers während der Aufnahme des zweiten Referenzpunktes verläuft.

Beschrieben wird weiter ein Computerprogramm, welches, wenn es in einen Computer geladen ist oder auf einem Computer läuft, Schritte des zuvor beschriebenen Verfahrens durchführt und ein Programmspeichermedium oder Computerprogrammprodukt mit diesem Computerprogramm.

Die erfindungsgemäße Vorrichtung zur Ermittlung der Stabilität und/oder Beweglichkeit eines Knies oder Kniegelenks eines Patienten umfasst ein Navigationssystem mit IR-Kameras, welche IR-Strahlung aussenden und reflektierte IR-Strahlung erfassen können oder welche emittierte IR-Strahlung erfassen können. Mit dem Navigationssystem ist eine Recheneinheit, insbesondere ein Computer oder ein Prozessor, drahtlos oder drahtgebunden verbunden oder die Recheneinheit ist in das Navigationssystem integriert, so dass Informationen zwischen dem Navigationssystem und der Recheneinheit ausgetauscht werden können. Werden mittels eines Pointers ein erster Referenzpunkt auf einer Tibia des Patienten und ein zweiter Referenzpunkt auf der Tibia oder der Patella des Patienten aufgenommen oder abgetastet, so kann das Navigationssystem die Position und/oder Orientierung des Pointers während des Aufnahme- oder Abtastvorgangs erfassen oder detektieren, indem die IR-Kameras die von einer an dem Pointer angeordneten Trackingreferenz reflektierte oder emittierte IR-Strahlung detektiert.

Die Recheneinheit kann aus der erfassten Position und/oder Orientierung des Pointers die Position des ersten und des zweiten Referenzpunkts bezüglich eines an der Tibia angeordneten ersten Koordinatensystems und/oder bezüglich eines an dem Femur angeordneten zweiten Koordinatensystems ermitteln. Sind die Positionen der beiden Referenzpunkte ermittelt und der Recheneinheit bekannt, kann die Recheneinheit die Position des ersten Referenzpunkts um einen vorgegebenen ersten Betrag, insbesondere um einen Betrag zwischen 1cm und 3cm, z.B. um 1.5cm, in einen ersten Achsenpunkt verschieben oder versetzen und die Position des zweiten Referenzpunkts um einen vorgegebenen zweiten Betrag, insbesondere um einen Betrag zwischen 2cm und 5cm, z.B. um 3cm, in einen zweiten Achsenpunkt versetzen.

Die Tibia und der Femur können nun in eine Position gebracht werden, in welcher sie sich relativ zueinander in vollständiger Streckung befinden, beispielsweise indem die Tibia und der Femur zumindest nahezu in einen Winkel von 180 Grad relativ zueinander gebracht werden. Wird in dieser Position ein Beweglichkeitstest zur Ermittlung der Beweglichkeit der Tibia relativ zu dem Femur durchgeführt, so kann die Recheneinheit bei der Durchführung des Beweglichkeitstests durch Ermittlung der Positionen und/oder Orientierungen eines ersten an der Tibia angeordneten Referenzsterns und eines zweiten an dem Femur angeordneten Referenzsterns die Positionen des ersten und des zweiten Achsenpunktes relativ zueinander während des Beweglichkeitstests ermitteln. Insbesondere werden die Positionen der Achsenpunkte zu Beginn des Beweglichkeitstests oder zu Beginn jedes Einzeltests des Beweglichkeitstests und bei maximaler Auslenkung ermittelt. Die ermittelten Positionen der Achsenpunkte während des Beweglichkeitstests können in der Recheneinheit z.B. als während des ersten Beweglichkeitstests gewonnene Positionen der Achsenpunkte zwischengespeichert werden, bei welchem die Tibia und der Femur vollkommen gestreckt relativ zueinander angeordnet waren.

Werden nun die Tibia und der Femur um einen ersten Winkelwert, insbesondere um 30 Grad, relativ zu der gestreckten Position abgewinkelt und sie befinden sich damit in einer um den ersten Winkelwert abgewinkelten Position relativ zu der gestreckten Position, wird ein weiterer oder zweiter Beweglichkeitstest durchgeführt. Die Recheneinheit kann bei der Durchführung des zweiten Beweglichkeitstests durch Ermittlung der Positionen und/oder Orientierungen des ersten und des zweiten Referenzsterns die Positionen des ersten und des zweiten Achsenpunktes relativ zueinander während des zweiten Beweglichkeitstests ermitteln und zwischenspeichern. Insbesondere kann die Recheneinheit die Start- und Endpositionen der Achsenpunkte während des zweiten Beweglichkeitstests ermitteln und zwischenspeichern.

Werden nun die Tibia und der Femur um einen vorgegebenen zweiten Winkelwert, insbesondere um 90 Grad, relativ zu der gestreckten Position relativ zueinander abgewinkelt, kann ein dritter oder ein weiterer Beweglichkeitstest durchgeführt werden. Die Recheneinheit kann bei der Durchführung des dritten Beweglichkeitstests durch Ermittlung der Positionen und/oder Orientierungen des ersten und des zweiten Referenzsterns die Positionen des ersten und des zweiten Achsenpunktes während des dritten Beweglichkeitstests ermitteln. Insbesondere kann die Recheneinheit die Start- und Endpositionen der Achsenpunkte während des dritten Beweglichkeitstests ermitteln und zwischenspeichern.

Vorzugsweise wird bei jedem der Beweglichkeitstests ein Rotationsbeweglichkeitstest, ein Translationsbeweglichkeitstest und ein Flexionsbeweglichkeitstest der Tibia und des Femurs relativ zueinander durchgeführt. Bei jedem der Rotationsbeweglichkeitstests, der Translationsbeweglichkeitstests und der Flexionsbeweglichkeitstests werden bevorzugt die Positionen der Achsenpunkte in der Ausgangsposition und in der Position maximaler Auslenkung, wie der maximalen Rotation der Tibia relativ zu dem Femur, der maximalen Translation der Tibia relativ zu dem Femur und der maximalen Flexion der Tibia relativ zu dem Femur, ermittelt. Aus den ermittelten Positionen der Achsenpunkte während der Beweglichkeitstests kann die Recheneinheit die Stabilität und Beweglichkeit des Knies oder Kniegelenks ermitteln oder ableiten.

Weiter kann die erfindungsgemäße Vorrichtung eine Anzeigevorrichtung, wie einen Bildschirm oder einen Monitor oder ein Display umfassen, welche mit dem Navigationssystem und/oder der Recheneinheit verbunden ist, und auf welcher die Tibia, der Femur, die Referenzpunkte und die Achsenpunkte und die weiteren ermittelten Daten dargestellt werden können.

Die Erfindung wird weiter anhand einer bevorzugten Ausführungsform beschrieben werden. Es zeigen:
- Figur 1a: eine Aufnahme eines ersten Referenzpunktes an einer Tibia eines Patienten mittels eines Pointers;
- Figur 1b: eine Aufnahme des ersten Referenzpunktes an der Tibia des Patienten bei einer um 180 Grad abgewinkelten Position der Tibia relativ zu einem Femur;
- Figur 2a: eine Aufnahme eines zweiten Referenzpunktes an einer Patella des Patienten mittels des Pointers bei der um 180 Grad abgewinkelten Position der Tibia relativ zu dem Femur in einer Draufsicht;
- Figur 2b: eine Aufnahme eines zweiten Referenzpunktes an einer Patella des Patienten mittels des Pointers bei der um 180 Grad abgewinkelten Position der Tibia relativ zu dem Femur in einer Seitenansicht;
- Figur 3a: die Ausgangsposition eines Beweglichkeitstests in einer Position, in welcher die Tibia relativ zu dem Femur um 30 Grad von einer vollständig gestreckten Position abgewinkelt ist;
- Figur 3b: die Durchführung eines Beweglichkeitstests in der in Figur 3a gezeigten Position der Tibia relativ zu dem Femur;
- Figur 4a: die Ausgangsposition eines Beweglichkeitstests in einer Position, in welcher die Tibia relativ zu dem Femur um 90 Grad von einer vollständig gestreckten Position abgewinkelt ist; und
- Figur 4b: die Durchführung eines Beweglichkeitstests in der in Figur 4a gezeigten Position der Tibia relativ zu dem Femur.

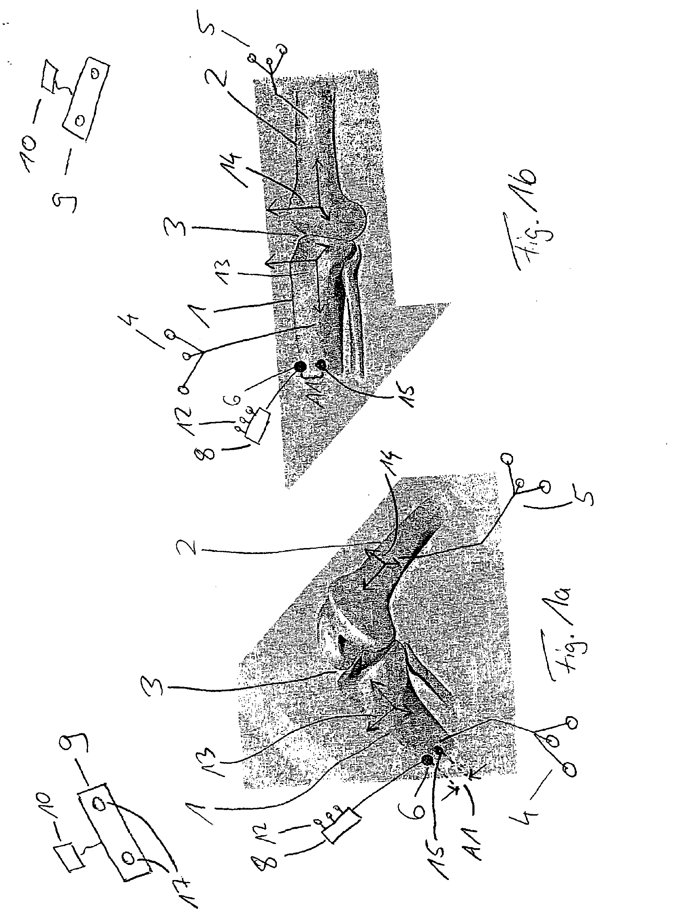

Die Figuren 1a und 1b zeigen einen Femur 2 und eine Tibia 1 eines Patienten, wobei sowohl an der Tibia 1 als auch an dem Femur 2 jeweils ein Referenzstern 4, 5 angeordnet ist. Mittels eines Pointers 8 wird als ein Referenzpunkt 6 ein Punkt an der anterioren Tibia 1 oder ein Punkt in einem anterior liegenden Bereich der Tibia 1 abgetastet oder aufgenommen. An dem Pointer 8 ist auch ein Referenzstern 12 angeordnet, so dass die Position und/oder Orientierung des an dem Pointer 8 angeordneten Referenzstern 12 mittels eines Navigationssystems 9 detektiert und ermittelt werden kann. Mit Hilfe der ermittelten Position und/oder Orientierung des an dem Pointer 8 angeordneten Referenzsterns 12 kann die Position und/oder Orientierung des Pointers 8 von dem Navigationssystem 9 ermittelt werden, so dass auch die Position und/oder Orientierung der Spitze des Pointers 8 von dem Navigationssystem 9 ermittelt werden kann. Somit kann die Position und/oder Orientierung der Spitze des Pointers 8 während des Abtast- oder Aufnahmevorganges des Referenzpunktes 6 auf der Tibia 1 von dem Navigationssystem 9 ermittelt werden, so dass auch die Position des Referenzpunktes 6 an der anterioren Tibia 1 von dem Navigationssystem 9 ermittelt werden kann. Somit ist sowohl die Position des Referenzpunkts 6 an der anterioren Tibia 1 als auch die Position und/oder Orientierung des Pointers 8, insbesondere der Spitze des Pointers 8, während des Aufnahmevorganges des Referenzpunkts 6 dem Navigationssystem bekannt 9. Aus der Position des Referenzpunkts 6 an der Tibia 1 kann das Navigationssystem 9, insbesondere eine mit dem Navigationssystem 9 verbundene Recheneinheit 10, einen Achsenpunkt 15 der Tibia 1 ermitteln, indem virtuell die Position des Referenzpunkts 6 um einen vorgegebenen ersten Betrag A1, wie z.B. um einen Betrag zwischen 0,5 und 2,5 cm, vorzugsweise um 1,5cm, posterior verschoben wird. Die Position des Referenzpunkts 6 wird um den vorgegebenen Betrag A1, z.B. um 1,5cm, posterior in eine Richtung verschoben, welche parallel zu der Orientierung des Pointers 8, insbesondere der Spitze des Pointers 8, während des Abtastvorganges des Referenzpunkts 6 ist, um den Achsenpunkt 15 der Tibia 1 zu ermitteln.

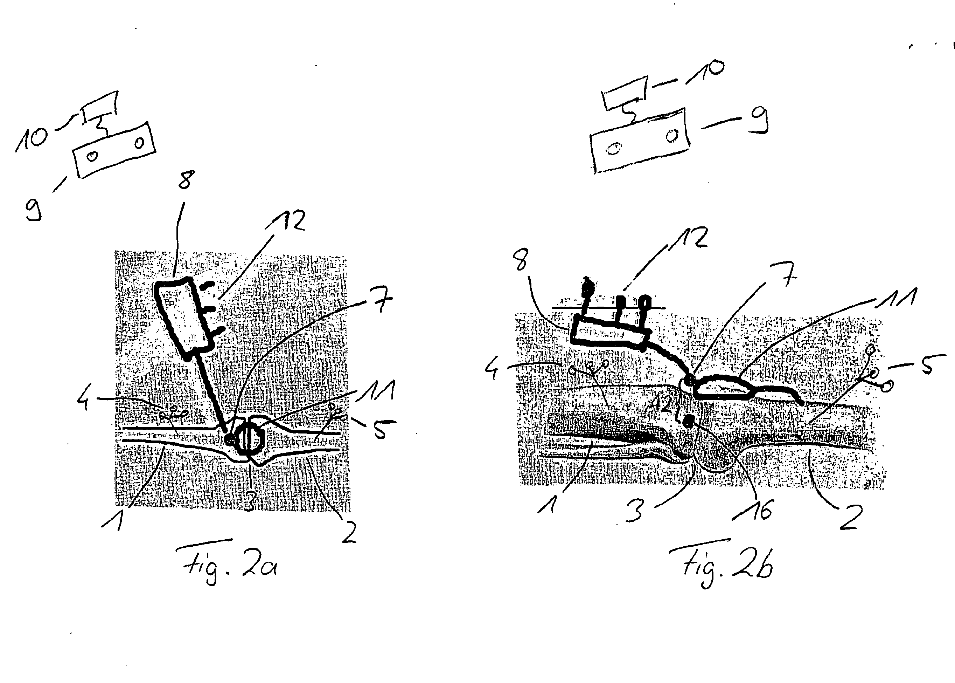

Die Figuren 2a und 2b zeigen, wie mittels des Pointers 8 ein Referenzpunkt 7 an dem inferioren Ende der Patella 11 (inferior patella pole) abgetastet oder aufgenommen wird. Auch die Position des Referenzpunktes 7 auf der Patella 11 kann von dem Navigationssystem 9, insbesondere von der mit dem Navigationssystem 9 verbundenen Recheneinheit 10, ermittelt werden, indem die Position und/oder Orientierung des Pointers 8, insbesondere der Spitze des Pointers 8, während des Aufnahmevorganges des an der Patella 11 angeordneten Referenzpunktes 7 mit Hilfe der Bestimmung der Position und/oder Orientierung des auf dem Pointer 8 angeordneten Referenzsterns 12 ermittelt wird. Während des Aufnahmevorganges des auf der Patella 11 liegenden Referenzpunktes 7 befindet sich die Tibia 1 vorzugsweise in vollständiger Streckung relativ zu dem Femur 2, so dass die Tibia 1 und der Femur 2 zumindest nahezu in einem Winkel von 180 Grad relativ zueinander liegen. Aus der ermittelten Position des auf der Patella 11 befindlichen Referenzpunktes 7 kann das Navigationssystem 9 einen weiteren Achsenpunkt 16 oder die Kniemitte ermitteln, indem das Navigationssystem 9 virtuell den Referenzpunkt 7 um einen vorgegebenen zweiten Betrag A2, wie um einen Wert zwischen 2 und 4 cm, insbesondere um 3cm, in das Knieinnere verschiebt. Der Referenzpunkt 7 wird posterior um den vorgegebenen zweiten Betrag A2 in eine Richtung verschoben, welche parallel zu der Orientierung ist, welche das Navigationssystem 9 als Orientierung des Pointers 8, insbesondere der Spitze des Pointers 8, während des Abtastens des auf der Patella 11 liegenden Referenzpunkts 7 ermittelt hat. Dadurch kann die Kniemitte des Knies 3 ermittelt werden. Sowohl der erste als auch der zweite Betrag A1, A2 sind abhängig von Patientenparametern, wie der Patientengröße oder der Knochendicke des Patienten. Bei einem Kind kann beispielsweise als erster Betrag A1 ein Wert von 0,5cm und ein zweiter Betrag A2 mit einem Wert von 1,5 oder 2cm verwendet werden, während bei einem großen, kräftigen Menschen als erster Betrag A1 ein Wert von 2cm oder mehr und als zweiter Betrag A2 ein Wert von 4cm oder mehr verwendet werden kann. Werden der Achsenpunkt der Tibia 1 und die Kniemitte in der Recheneinheit 10 miteinander virtuell verbunden erhält man eine Approximation oder Näherung der mechanischen Tibiaachse. Beispielsweise durch Spiegelung an der Kniemitte kann die Recheneinheit 10 aus der approximierten Tibiaachse auch eine Näherung der Femurachse ermitteln.

In der Position, in welcher die Tibia 1 und der Femur 2 vollständig gestreckt relativ zueinander angeordnet sind oder um 180 Grad relativ zueinander gestreckt sind, wird ein erster Beweglichkeitstest durchgeführt. Der Beweglichkeitstest umfasst einen Rotations-, einen Translations- und einen Flexionstest, bei welchen jeweils die Tibia 1 relativ zu dem Femur 2 maximal ausgelenkt wird. Vor Beginn des Beweglichkeitstests wird die Tibia 1 in eine neutrale Rotationsposition relativ zu dem Femur 2 gebracht, so dass die Tibia 1 relativ zu dem Femur 2 nicht verdreht ist. Bei dem Beweglichkeitstest wird zu Beginn die Position der Achsenpunkte 15, 16 und/oder der Tibiaachse und/oder der Femurachse bestimmt. Bei jedem der Beweglichkeitstests wird die Tibia 1 relativ zu dem Femur 2 maximal ausgelenkt und es wird die Position der Achsenpunkte 15, 16 und/oder der Tibiaachse und/oder der Femurachse bei der maximalen Auslenkung oder in dem Punkt maximaler Auslenkung bestimmt. Bei dem Rotationsbeweglichkeitstest wird die Tibia 1 relativ zu dem Femur 2 rotiert oder gedreht bis eine maximale Rotationsauslenkung erreicht ist und in dem Punkt maximaler Rotationsauslenkung wird die Position der Achsenpunkte 15, 16 und/oder der Tibiaachse und/oder der Femurachse bestimmt. Bei dem Translationsbeweglichkeitstest wird die Tibia 1 relativ zu dem Femur 2 verschoben bis eine maximale Translationsauslenkung erreicht ist und in dem Punkt maximaler Translationsauslenkung wird die Position der Achsenpunkte 15, 16 und/oder der Tibiaachse und/oder der Femurachse bestimmt. Bei dem Flexionsbeweglichkeitstest wird die Tibia 1 relativ zu dem Femur 2 gestreckt oder gebeugt bis eine maximale Flexionssauslenkung erreicht ist und in dem Punkt maximaler Flexionsauslenkung wird die Position der Achsenpunkte 15, 16 und/oder der Tibiaachse und/oder der Femurachse bestimmt. Damit erhält man mit dem ersten Beweglichkeitstest eine Vielzahl von Positionen der Achsenpunkte 15, 16 und/oder der Tibiaachse und/oder der Femurachse, welche beispielsweise zwischengespeichert werden.

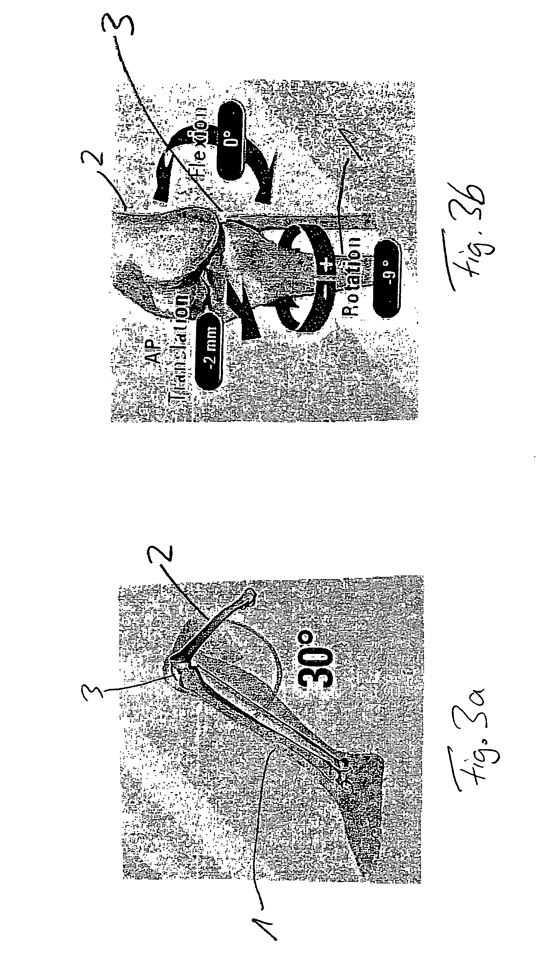

Nach der Durchführung des ersten Beweglichkeitstests wird die Tibia 1, wie in Figur 3a gezeigt, aus der gestreckten Ausgangslage relativ zu dem Femur 2 um einen vorgegebenen ersten Betrag, insbesondere um 30 Grad, abgewinkelt. In dieser Position wird, wie in Figur 3b gezeigt, ein zweiter Beweglichkeitstest, welcher genauso ablaufen kann wie der erste Beweglichkeitstest und welcher ebenfalls einen Rotationsbeweglichkeitest, einen Translationsbeweglichkeitstest und einen Flexionsbeweglichkeitstest umfassen kann, durchgeführt. Auch während des zweiten Beweglichkeitstest werden vorzugsweise die Positionen der Achsenpunkte 15, 16 und/oder der Tibiaachse und/oder der Femurachse in einer Startposition des Beweglichkeitstest und in einer Position maximaler Auslenkung ermittelt. Daraus ergeben sich eine Vielzahl von Positionen der Achsenpunkte 15, 16 und/oder der Tibiaachse und/oder der Femurachse, welche beispielsweise zwischengespeichert werden.

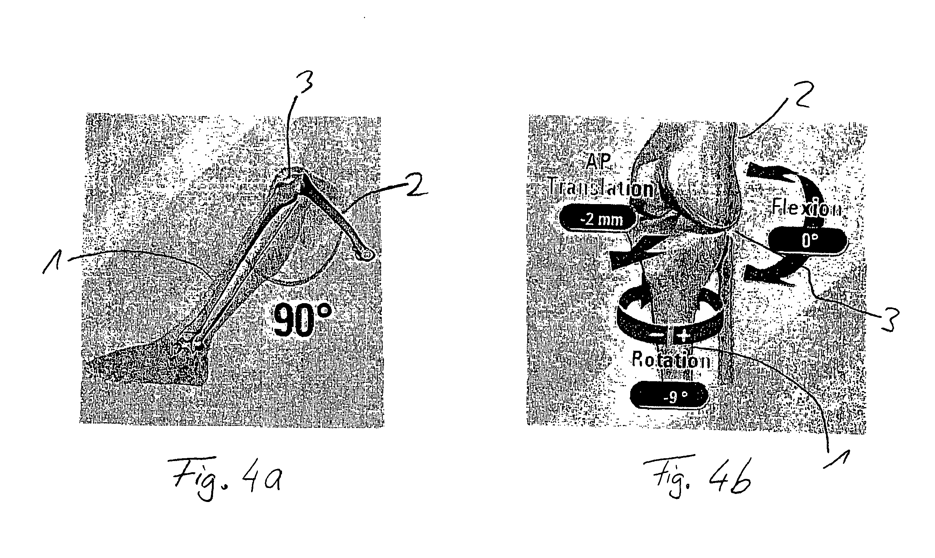

Nach der Durchführung des zweiten Beweglichkeitstests wird die Tibia 1 aus der um 30 Grad von der gestreckten Ausgangslage relativ zu dem Femur 2 abweichenden Position um einen vorgegebenen zweiten Betrag, insbesondere um weitere 60 Grad, abgewinkelt, so dass die Tibia 1 und der Femur 2, wie in Figur 4a gezeigt, in einem Winkel von 90 Grad bezüglich der gestreckten Position relativ zueinander angeordnet sind. In dieser Position wird, wie in Figur 4b gezeigt, ein dritter Beweglichkeitstest, welcher genauso ablaufen kann wie der erste und/oder der zweite Beweglichkeitstest und welcher ebenfalls einen Rotationsbeweglichkeitest, einen Translationsbeweglichkeitstest und einen Flexionsbeweglichkeitstest umfassen kann, durchgeführt. Auch während des dritten Beweglichkeitstests werden vorzugsweise die Positionen der Achsenpunkte 15, 16 und/oder der Tibiaachse und/oder der Femurachse in einer Startposition des Beweglichkeitstests und in einer Position maximaler Auslenkung ermittelt. Daraus ergeben sich eine Vielzahl von Positionen der Achsenpunkte 15, 16 und/oder der Tibiaachse und/oder der Femurachse, welche beispielsweise zwischengespeichert werden.

Zumindest aus einem Teil der ermittelten Positionen oder aus all den ermittelten Positionen der Achsenpunkte 15, 16 und/oder der Tibiaachse und/oder der Femurachse während der Beweglichkeitstests kann das Navigationssystem 9 die Stabilität oder die Beweglichkeit des Knies 3 oder des Kniegelenks oder des Kreuzbandes ermitteln. Die ermittelte Stabilität oder Beweglichkeit des Kniegelenks kann z.B. vor einer Kniegelenks-Operation ermittelt werden und kann verwendet werden, um während oder nach einer Kniegelenks-Operation die bisherige oder ursprünglich bestehende Stabilität oder Beweglichkeit des Knies 3 oder Kniegelenks wiederherzustellen oder zu erhalten oder um zu überprüfen, wie stark die Stabilität oder Beweglichkeit nach der Operation von der ursprünglichen Stabilität oder Beweglichkeit vor der Operation abweicht.

## Patentansprüche

1. Verfahren zur Ermittlung der Stabilität und Beweglichkeit eines Knies (3) oder Kniegelenks eines Patienten, wobei an der Tibia (1) des Patienten ein erster Referenzstern (4) angeordnet ist und an dem Femur (2) des Patienten ein zweiter Referenzstern (5) angeordnet ist, wobei mittels eines navigierbaren Instruments, insbesondere eines Pointers (8), ein erster Referenzpunkt (6) auf oder über der Tibia (1) aufgenommen wird und mittels des navigierbaren Instruments oder Pointers (8) ein zweiter Referenzpunkt (7) auf oder über der Tibia (1) oder der Patella (11) aufgenommen wird, die Position und/oder Orientierung des navigierbaren Instruments oder Pointers (8) während des Aufnahmevorganges des ersten und des zweiten Referenzpunktes (6, 7) mit Hilfe einer an dem navigierbaren Instrument oder Pointer (8) angeordneten Trackingreferenz (12) von einem Navigationssystem (9) erfasst und ermittelt wird, wobei aus der Position und/oder Orientierung des navigierbaren Instruments oder Pointers (8) die Position des ersten und des zweiten Referenzpunkts (6, 7) bezüglich eines an der Tibia (1) angeordneten ersten Koordinatensystems (13) und/oder bezüglich eines an dem Femur (2) angeordneten zweiten Koordinatensystems (14) ermittelt wird, wobei die Position des ersten Referenzpunkts (6) um einen vorgegebenen ersten Betrag (A1) parallel zu der Pointer-Richtung, insbesondere zwischen 1cm und 3cm, z.B. 1.5cm, in einen ersten Achsenpunkt (15) versetzt wird und die Position des zweiten Referenzpunkts (7) um einen vorgegebenen zweiten Betrag (A2) parallel zu der Pointer-Richtung, insbesondere zwischen 2cm und 5cm, z.B. 3cm, in einen zweiten Achsenpunkt (16) versetzt wird, um die mechanische Tibiaachse zu aproximieren, wobei die Tibia (1) und der Femur (2) zumindest nahezu in einen Winkel von 180 Grad relativ zueinander in eine gestreckte Position gebracht werden, durch Spiegelungen an der Kniemitte aus der approximierten Tibiaachse eine Näherung der Femurachse ermittelt wird und in welcher ein Beweglichkeitstest zur Ermittlung der Beweglichkeit der Tibia (1) relativ zu dem Femur (2) durchgeführt wird und mittels des Navigationssystems (9) durch Ermittlung der Positionen und/oder Orientierungen des ersten und des zweiten Referenzsterns (4,5) die Positionen der Tibiaachse und der Femurachse relativ zueinander während des Beweglichkeitstests bestimmt werden, die Tibia (1) und der Femur (2) um einen vorgegebenen ersten Winkelwert, insbesondere um 30 Grad, relativ zu der gestreckten Position abgewinkelt werden, in dieser Position der Beweglichkeitstest durchgeführt wird und mittels des Navigationssystems (9) durch Ermittlung der Positionen und/oder Orientierungen des ersten und des zweiten Referenzsterns (4, 5) die Positionen der Tibiaachse und der Femurachse relativ zueinander während des Beweglichkeitstests ermittelt werden, die Tibia (1) und der Femur (2) um einen vorgegebenen zweiten Winkelwert, insbesondere um 90 Grad, relativ zu der gestreckten Position abgewinkelt werden, in dieser Position der Beweglichkeitstest durchgeführt wird und mittels des Navigationssystems (9) durch Ermittlung der Positionen und/oder Orientierungen des ersten und des zweiten Referenzsterns (4, 5) die Positionen der Tibiaachse und der Femurachse während des Beweglichkeitstests ermittelt werden, und aus den ermittelten Positionen der Achsenpunkte (15, 16) die Stabilität und Beweglichkeit des Knies (3) oder Kniegelenks ermittelt wird.

2. Verfahren nach einem der vorhergehenden Ansprüche, wobei als erster Referenzpunkt (6) ein Punkt auf der anterioren Tibia (1) verwendet wird.

3. Verfahren nach einem der vorhergehenden Ansprüche, wobei als zweiter Referenzpunkt (7) ein Punkt auf dem inferioren Ende der Patella (Im Englischen: patella pole) verwendet wird.

4. Verfahren nach dem vorhergehenden Anspruch, wobei durch Verbindung des ersten und des zweiten Achsenpunkts (15, 16) die Tibiaachse approximiert wird.

5. Verfahren nach dem vorhergehenden Anspruch, wobei in der gestreckten Position der Tibia (1) und des Femurs (2) relativ zueinander, in der um 30 Grad von der gestreckten Position abgewinkelten Position und in der um 90 Grad von der gestreckten Position abgewinkelten Position mittels des Navigationssystems (9) durch Ermittlung der Positionen und/oder Orientierungen des ersten und des zweiten Referenzsterns (4, 5) die Positionen und/oder Orientierungen der Tibiaachse während der Beweglichkeitstests ermittelt werden.

6. Verfahren nach einem der zwei vorhergehenden Ansprüche, wobei aus der Tibiaachse durch Transformation oder Spiegelung, insbesondere an dem zweiten Achsenpunkt (16), z.B. dem Kniemittelpunkt, die Femurachse approximiert wird.

7. Verfahren nach einem der vorhergehenden Ansprüche, wobei als Beweglichkeitstest ein Rotationsbeweglichkeitstest, ein Translationsbeweglichkeitstest und ein Flexionsbeweglichkeitstest der Tibia (1) und des Femurs (2) relativ zueinander durchgeführt wird.

8. Verfahren nach dem vorhergehenden Anspruch, wobei bei dem Rotationsbeweglichkeitstest durch Ermittlung der Positionen des ersten Achsenpunkts (15) relativ zu dem zweiten Achsenpunkt (16) ermittelt wird, um welchen Rotationswinkel die Tibia (1) relativ zu dem Femur (2) gedreht werden kann.

9. Verfahren nach einem der zwei vorhergehenden Ansprüche, wobei bei dem Translationsbeweglichkeitstest durch Ermittlung der Positionen des ersten Achsenpunkts (15) relativ zu dem zweiten Achsenpunkt (16) ermittelt wird, um welchen Translationsbetrag die Tibia (1) relativ zu dem Femur (2) in anteriore Richtung verschoben werden kann.

10. Verfahren nach einem der drei vorhergehenden Ansprüche, wobei bei dem Flexionsbeweglichkeitstest durch Ermittlung der Positionen des ersten Achsenpunkts (15) relativ zu dem zweiten Achsenpunkt (16) ermittelt wird, um welchen Flexionswinkel die Tibia (1) relativ zu dem Femur (2) gebeugt werden kann

11. Verfahren nach einem der vorhergehenden Ansprüche, wobei vor den Beweglichkeitstests die Tibia (1) und der Femur (2) in eine neutrale Rotationsposition relativ zueinander gebracht werden.

12. Verfahren nach einem der vorhergehenden Ansprüche, wobei als Ursprung des ersten und des zweiten Koordinatensystems (13, 14) der zweite Achsenpunkt (16), insbesondere die Kniemitte, verwendet wird.

13. Verfahren nach einem der vorhergehenden Ansprüche, wobei als eine Achse des ersten Koordinatensystems (13) die Tibiachse verwendet wird und als eine weitere Achse des ersten Koordinatensystems (13) eine Achse verwendet wird, welche parallel zu der Orientierung des Pointers (8) während der Aufnahme des ersten Referenzpunktes (6) verläuft.

14. Verfahren nach einem der vorhergehenden Ansprüche, wobei als eine Achse des zweiten Koordinatensystems (14) die Femurachse verwendet wird und als eine weitere Achse des zweiten Koordinatensystems (14) eine Achse verwendet wird, welche parallel zu der Orientierung des Pointers (8) während der Aufnahme des zweiten Referenzpunktes (7) verläuft.

15. Vorrichtung zur Ermittlung der Stabilität und Beweglichkeit eines Knies (3) oder Kniegelenks eines Patienten umfassend ein Navigationssystem (9) mit IR-Kameras (17) und eine mit dem Navigationssystem (9) verbundene oder in das Navigationssystem (9) integrierte Recheneinheit (10), wobei das Navigationssystem (9) die Position und/oder Orientierung eines Pointers (8) während des Aufnahmevorganges eines ersten Referenzpunkts (6) auf einer Tibia (1) und eines zweiten Referenzpunktes (7) auf der Tibia (1) oder der Patella (11) mit Hilfe einer an dem Pointer (8) angeordneten Trackingreferenz (12) erfassen kann, wobei die Recheneinheit (10) aus der erfassten Position und/oder Orientierung des Pointers (8) die Position des ersten und des zweiten Referenzpunkts (6, 7) bezüglich eines an der Tibia (1) angeordneten ersten Koordinatensystems (13) und/oder bezüglich eines an dem Femur (2) angeordneten zweiten Koordinatensystems (14) ermitteln kann, die Position des ersten Referenzpunkts (6) um einen vorgegebenen ersten Betrag (A1) parallel zu der Pointer-Richtung, insbesondere zwischen 1cm und 3cm, z.B. 1.5cm, in einen ersten Achsenpunkt (15) versetzen kann und die Position des zweiten Referenzpunkts (7) um einen vorgegebenen zweiten Betrag (A2) parallel zu der Pointer-Richtung, insbesondere zwischen 2cm und 5cm, z.B. 3cm, in einen zweiten Achsenpunkt (16) versetzen kann, um die mechanische Tibiaachse zu approximieren, wobei die Recheneinheit (10), wenn sich die Tibia (1) und der Femur (2) zumindest nahezu in einen Winkel von 180 Grad relativ zueinander in einer gestreckte Position befinden, durch Spiegelungen an der Kniemitte aus der approximierten Tibiaachse eine Näherung der Femurachse ermittelt und bei der Durchführung eines Beweglichkeitstests zur Ermittlung der Beweglichkeit der Tibia (1) relativ zu dem Femur (2) durch Ermittlung der Positionen und/oder Orientierungen eines ersten an der Tibia (1) angeordneten Referenzsterns (4) und eines zweiten an dem Femur (2) angeordneten Referenzsterns (5) die Positionen der Tibiaachse und der Femurachse relativ zueinander während des Beweglichkeitstests ermitteln kann, wobei die Recheneinheit (10), wenn sich die Tibia (1) und der Femur (2) in einer um einen vorgegebenen ersten Winkelwert, insbesondere um 30 Grad, relativ zu der gestreckten Position abgewinkelten Position befinden, bei der Durchführung des Beweglichkeitstests durch Ermittlung der Positionen und/oder Orientierungen des ersten und des zweiten Referenzsterns (4, 5) die Positionen der Tibiaachse und der Femurachse relativ zueinander während des Beweglichkeitstests ermitteln kann, wobei die Recheneinheit (10), wenn sich die Tibia (1) und der Femur (2) in einer um einen vorgegebenen zweiten Winkelwert, insbesondere um 90 Grad, relativ zu der gestreckten Position abgewinkelten Position befinden, bei der Durchführung des Beweglichkeitstests durch Ermittlung der Positionen und/oder Orientierungen des ersten und des zweiten Referenzsterns (4, 5) die Positionen der Tibiaachse und der Femurachse während des Beweglichkeitstests ermitteln kann, und wobei die Recheneinheit (10) aus den ermittelten Positionen der Achsenpunkte die Stabilität und Beweglichkeit des Knies (3) oder Kniegelenksermitteln kann.

16. Vorrichtung nach dem vorhergehenden Anspruch, wobei die Vorrichtung weiter eine Anzeigevorrichtung umfasst, welche mit dem Navigationssystem (9) und/oder der Recheneinheit (10) verbunden ist, und auf welcher die Tibia (1), der Femur (2), die Referenzpunkte (6, 7) und die Achsenpunkte (15, 16) dargestellt werden können.

## Claims

1. A method for ascertaining the stability and mobility of a knee (3) or knee joint of a patient, wherein: a first reference star (4) is arranged on the tibia (1) of the patient and a second reference star (5) is arranged on the femur (2) of the patient; a first reference point (6) on or over the tibia (1) is recorded by means of a navigable instrument, in particular a pointer (8), and a second reference point (7) on or over the tibia (1) or the patella (11) is recorded by means of the navigable instrument or pointer (8); a navigation system (9) detects and ascertains the position and/or orientation of the navigable instrument or pointer (8) during the recording process of the first and second reference point (6, 7) with the aid of a tracking reference (12) arranged on the navigable instrument or pointer (8); the position of the first and second reference point (6, 7) with respect to a first co-ordinate system (13) arranged on the tibia (1) and/or with respect to a second co-ordinate system (14) arranged on the femur (2) is ascertained from the position and/or orientation of the navigable instrument or pointer (8); the position of the first reference point (6) is offset parallel to the pointer direction by a first predetermined amount (A1), in particular between 1 and 3 cm, for example 1.5 cm, into a first axial point (15), and the position of the second reference point (7) is offset parallel to the pointer direction by a second predetermined amount (A2), in particular between 2 and 5 cm, for example 3 cm, into a second axial point (16), in order to approximate the mechanical tibia axis; the tibia (1) and the femur (2) are placed in an extended position at least almost at an angle of 180° relative to each other, an approximation of the femur axis is ascertained from the approximated tibia axis by reflecting it at the centre of the knee, a mobility test is performed in the extended position in order to ascertain the mobility of the tibia (1) relative to the femur (2), and the navigation system (9) determines the positions of the tibia axis and the femur axis relative to each other during the mobility test by ascertaining the positions and/or orientations of the first and second reference star (4, 5); the tibia (1) and the femur (2) are bent by a predetermined first angular value, in particular by 30°, relative to the extended position; the mobility test is performed in this position, and the navigation system (9) ascertains the positions of the tibia axis and the femur axis relative to each other during the mobility test by ascertaining the positions and/or orientations of the first and second reference star (4, 5); the tibia (1) and the femur (2) are bent by a predetermined second angular value, in particular by 90°, relative to the extended position; the mobility test is performed in this position, and the navigation system (9) ascertains the positions of the tibia axis and the femur axis during the mobility test by ascertaining the positions and/or orientations of the first and second reference star (4, 5); and the stability and mobility of the knee (3) or knee joint is ascertained from the ascertained positions of the axial points (15, 16).

2. The method according to the preceding claim, wherein a point on the anterior tibia (1) is used as the first reference point (6).

3. The method according to any one of the preceding claims, wherein a point on the inferior end of the patella (the patella pole) is used as the second reference point (7).

4. The method according to the preceding claim, wherein the tibia axis is approximated by connecting the first and second axial point (15, 16).

5. The method according to the preceding claim, wherein in the extended position of the tibia (1) and the femur (2) relative to each other, the position bent from the extended position by 30°, and the position bent from the extended position by 90°, the navigation system (9) ascertains the positions and/or orientations of the tibia axis during the mobility tests by ascertaining the positions and/or orientations of the first and second reference star (4, 5).

6. The method according to any one of the preceding two claims, wherein the femur axis is approximated from the tibia axis by transforming or reflecting the tibia axis, in particular at the second axial point (16), for example the centre point of the knee.

7. The method according to any one of the preceding claims, wherein a rotational mobility test, a translational mobility test and a flexion mobility test of the tibia (1) and the femur (2) relative to each other are performed as the mobility test.

8. The method according to the preceding claim, wherein in the rotational mobility test, the rotational angle by which the tibia (1) can be rotated relative to the femur (2) is ascertained by ascertaining the positions of the first axial point (15) relative to the second axial point (16).

9. The method according to any one of the preceding two claims, wherein in the translational mobility test, the translational amount by which the tibia (1) can be shifted relative to the femur (2) in the anterior direction is ascertained by ascertaining the positions of the first axial point (15) relative to the second axial point (16).

10. The method according to any one of the preceding three claims, wherein in the flexion mobility test, the flexion angle by which the tibia (1) can be flexed relative to the femur (2) is ascertained by ascertaining the positions of the first axial point (15) relative to the second axial point (16).

11. The method according to any one of the preceding claims, wherein the tibia (1) and the femur (2) are placed in a neutral rotational position relative to each other before the mobility tests.

12. The method according to any one of the preceding claims, wherein the second axial point (16), in particular the centre of the knee, is used as the origin of the first and second co-ordinate system (13, 14).

13. The method according to any one of the preceding claims, wherein the tibia axis is used as an axis of the first co-ordinate system (13), and an axis which runs parallel to the orientation of the pointer (8) while recording the first reference point (6) is used as another axis of the first co-ordinate system (13).

14. The method according to any one of the preceding claims, wherein the femur axis is used as an axis of the second co-ordinate system (14), and an axis which runs parallel to the orientation of the pointer (8) while recording the second reference point (7) is used as another axis of the second co-ordinate system (14).

15. A device for ascertaining the stability and mobility of a knee (3) or knee joint of a patient, comprising a navigation system (9) having infrared cameras (17), and a computational unit (10) which is connected to the navigation system (9) or is integrated into the navigation system (9), wherein: the navigation system (9) can detect the position and/or orientation of a pointer (8) during the recording process of a first reference point (6) on a tibia (1) and a second reference point (7) on the tibia (1) or the patella (11) with the aid of a tracking reference (12) arranged on the pointer (8); the computational unit (10) can ascertain the position of the first and second reference point (6, 7) with respect to a first co-ordinate system (13) arranged on the tibia (1) and/or with respect to a second co-ordinate system (14) arranged on the femur (2) from the detected position and/or orientation of the pointer (8), offset the position of the first reference point (6) by a first predetermined amount (A1) parallel to the pointer direction, in particular between 1 and 3 cm, for example 1.5 cm, into a first axial point (15), and offset the position of the second reference point (7) by a second predetermined amount (A2) parallel to the pointer direction, in particular between 2 and 5 cm, for example 3 cm, into a second axial point (16), in order to approximate the mechanical tibia axis; when the tibia (1) and the femur (2) are situated in an extended position at least almost at an angle of 180° relative to each other, the computational unit (10) ascertains an approximation of the femur axis from the approximated tibia axis by reflecting it at the centre of the knee, and when a mobility test is performed in order to ascertain the mobility of the tibia (1) relative to the femur (2), the computational unit (10) can ascertain the positions of the tibia axis and the femur axis relative to each other during the mobility test by ascertaining the positions and/or orientations of a first reference star (4) arranged on the tibia (1) and a second reference star (5) arranged on the femur (2); when the tibia (1) and the femur (2) are situated in a position bent by a predetermined first angular value, in particular by 30°, relative to the extended position and when the mobility test is performed, the computational unit (10) can ascertain the positions of the tibia axis and the femur axis relative to each other during the mobility test by ascertaining the positions and/or orientations of the first and second reference star (4, 5); when the tibia (1) and the femur (2) are situated in a position bent by a predetermined second angular value, in particular by 90°, relative to the extended position and when the mobility test is performed, the computational unit (10) can ascertain the positions of the tibia axis and the femur axis during the mobility test by ascertaining the positions and/or orientations of the first and second reference star (4, 5); and the computational unit (10) can ascertain the stability and mobility of the knee (3) or knee joint from the ascertained positions of the axial points.

16. The device according to the preceding claim, wherein the device also comprises a display device which is connected to the navigation system (9) and/or the computational unit (10) and on which the tibia (1), the femur (2), the reference points (6, 7) and the axial points (15, 16) can be displayed.

## Revendications

1. Procédé pour déterminer la stabilité et la mobilité du genou (3) ou de l'articulation du genou d'un patient, dans lequel première étoile de référence (4) est disposée sur le tibia (1) du patient et une deuxième étoile de référence (5) est disposée sur le fémur (2) du patient, dans lequel au moyen d'un instrument navigable, notamment d'un pointeur (8), on enregistre un premier point de référence (6) sur ou au-dessus du tibia (1) et qu'au moyen de l'instrument navigable ou pointeur (8), on enregistre un deuxième point de référence (7) sur ou au-dessus du tibia (1) ou de la rotule (11), et que la position et/ou l'orientation de l'instrument navigable ou pointeur (8) pendant le processus d'enregistrement du premier et du deuxième points de référence (6, 7) est détectée et déterminée par un système de navigation (9) à l'aide d'une référence de poursuite (12) disposée sur l'instrument navigable ou pointeur (8), dans lequel à partir de la position et/ou de l'orientation de l'instrument navigable ou pointeur (8), on détermine la position du premier et du deuxième points de référence (6, 7) par rapport à un premier système de coordonnées (13) disposé sur le tibia (1) et/ou par rapport à un deuxième système de coordonnées (14) disposé sur le fémur (2), dans lequel la position du premier point de référence (6) est décalée d'une première valeur prédéterminée (A1) parallèlement à la direction du pointeur, notamment d'entre 1 cm et 3 cm, par exemple de 1,5 cm, en un premier point axial (15), et que la position du deuxième point de référence (7) est décalée d'une deuxième valeur prédéterminée (A2) parallèlement à la direction du pointeur, notamment d'entre 2 cm et 5 cm, par exemple de 3 cm, en un deuxième point axial (16), afin d'obtenir une approximation de l'axe mécanique du tibia, dans lequel le tibia (1) et le fémur (2) sont amenés dans une position déployée sous un angle d'au moins à peu près 180 degrés l'un par rapport à l'autre, et qu'on détermine une approximation de l'axe du fémur à partir de l'approximation de l'axe du tibia par des réflexions sur le centre du genou, et dans lequel procédé on effectue un test de mobilité pour déterminer la mobilité du tibia (1) par rapport au fémur (2) et, au moyen du système de navigation (9), en déterminant les positions et/ou orientations de la première et de la deuxième étoiles de référence (4, 5), on détermine les positions de l'axe du tibia et de l'axe du fémur l'un par rapport à l'autre pendant le test de mobilité, on plie le tibia (1) et le fémur (2) d'une première valeur d'angle prédéterminée, notamment de 30 degrés, par rapport à la position déployée, on effectue le test de mobilité dans cette position et, au moyen du système de navigation (9), en déterminant les positions et/ou orientations de la première et de la deuxième étoiles de référence (4, 5), on détermine les positions de l'axe du tibia et de l'axe du fémur l'un par rapport à l'autre pendant le test de mobilité, on plie le tibia (1) et le fémur (2) d'une deuxième valeur d'angle prédéterminée, notamment de 90 degrés, par rapport à la position déployée, on effectue le test de mobilité dans cette position et, au moyen du système de navigation (9), en déterminant les positions et/ou orientations de la première et de la deuxième étoiles de référence (4, 5), on détermine les positions de l'axe du tibia et de l'axe du fémur pendant le test de mobilité, et à partir des positions déterminées des points axiaux (15, 16), on détermine la stabilité et la mobilité du genou (3) ou de l'articulation du genou.

2. Procédé selon la revendication précédente, dans lequel on utilise comme premier point de référence (6) un point sur le tibia antérieur (1).

3. Procédé selon l'une des revendications précédentes, dans lequel on utilise comme deuxième point de référence (7) un point sur l'extrémité inférieure de la rotule (en anglais : « patella pole »).

4. Procédé selon la revendication précédente, dans lequel on obtient une approximation de l'axe du tibia en reliant le premier point axial (15) et le deuxième point axial (16).

5. Procédé selon la revendication précédente, dans lequel, dans la position déployée du tibia (1) et du fémur (2) l'un par rapport à l'autre, dans la position pliée de 30 degrés par rapport à la position déployée et dans la position pliée de 90 degrés par rapport à la position déployée, on détermine au moyen du système de navigation (9) les positions et/ou orientations de l'axe du tibia pendant le test de mobilité en déterminant les positions et/ou orientations de la première et de la deuxième étoiles de référence (4, 5).

6. Procédé selon l'une des deux revendications précédentes, dans lequel on obtient une approximation de l'axe du fémur à partir de l'axe du tibia par transformation ou réflexion, notamment sur le deuxième point axial (16), par exemple sur le centre du genou.

7. Procédé selon l'une des revendications précédentes, dans lequel on effectue comme test de mobilité un test de mobilité en rotation, un test de mobilité en translation et un test de mobilité en flexion du tibia (1) et du fémur (2) l'un par rapport à l'autre.

8. Procédé selon la revendication précédente, dans lequel, lors du test de mobilité en rotation, en déterminant les positions du premier point axial (15) par rapport au deuxième point axial (16), on détermine de quel angle de rotation le tibia (1) peut être tourné par rapport au fémur (2).

9. Procédé selon l'une des deux revendications précédentes, dans lequel, lors du test de mobilité en translation, en déterminant les positions du premier point axial (15) par rapport au deuxième point axial (16), on détermine de quel montant de translation le tibia (1) peut être déplacé par rapport au fémur (2) en direction antérieure.

10. Procédé selon l'une des trois revendications précédentes, dans lequel, lors du test de mobilité en flexion, en déterminant les positions du premier point axial (15) par rapport au deuxième point axial (16), on détermine de quel angle de flexion le tibia (1) peut être plié par rapport au fémur (2).

11. Procédé selon l'une des revendications précédentes, dans lequel avant les tests de mobilité, le tibia (1) et le fémur (2) sont amenés dans une position neutre de rotation l'un par rapport à l'autre.

12. Procédé selon l'une des revendications précédentes, dans lequel on utilise comme origine du premier et du deuxième systèmes de coordonnées (13, 14) le deuxième point axial (16), notamment le centre du genou.

13. Procédé selon l'une des revendications précédentes, dans lequel on utilise comme premier axe du premier système de coordonnées (13) l'axe du tibia, et comme autre axe du premier système de coordonnées (13) un axe qui s'étend parallèlement à l'orientation du pointeur (8) pendant l'enregistrement du premier point de référence (6).

14. Procédé selon l'une des revendications précédentes, dans lequel on utilise comme premier axe du deuxième système de coordonnées (14) l'axe du fémur, et comme autre axe du deuxième système de coordonnées (14) un axe qui s'étend parallèlement à l'orientation du pointeur (8) pendant l'enregistrement du deuxième point de référence (7).

15. Dispositif pour déterminer la stabilité et la mobilité du genou (3) ou de l'articulation du genou d'un patient, comprenant un système de navigation (9) avec des caméras infrarouges (17) et une unité de calcul (10) reliée au système de navigation (9) ou intégrée dans le système de navigation (9), dans lequel le système de navigation (9) peut détecter la position et/ou l'orientation d'un pointeur (8) pendant le processus d'enregistrement d'un premier point de référence (6) sur un tibia (1) et d'un deuxième point de référence (7) sur le tibia (1) ou la rotule (11) à l'aide d'une référence de poursuite (12) disposée sur le pointeur (8), dans lequel à partir de la position et/ou de l'orientation détectée du pointeur (8), l'unité de calcul (10) peut déterminer la position du premier et du deuxième points de référence (6, 7) par rapport à un premier système de coordonnées (13) disposé sur le tibia (1) et/ou par rapport à un deuxième système de coordonnées (14) disposé sur le fémur (2), que la position du premier point de référence (6) peut être décalée d'une premier valeur prédéterminée (A1) parallèlement à la direction du pointeur, notamment d'entre 1 cm et 3 cm, par exemple de 1,5 cm, en un premier point axial (15), et que la position du deuxième point de référence (7) peut être décalée d'une deuxième valeur prédéterminée (A2) parallèlement à la direction du pointeur, notamment d'entre 2 cm et 5 cm, par exemple de 3 cm, en un deuxième point axial (16), afin d'obtenir une approximation de l'axe mécanique du tibia, dans lequel l'unité de calcul (10), lorsque le tibia (1) et le fémur (2) se trouvent dans une position déployée sous un angle d'au moins à peu près 180 degrés l'un par rapport à l'autre, détermine une approximation de l'axe du fémur à partir de l'approximation de l'axe du tibia par des réflexions sur le centre du genou et, lors de l'exécution d'un test de mobilité pour déterminer la mobilité du tibia (1) par rapport au fémur (2), en déterminant les positions et/ou orientations d'une première étoile de référence (4) disposée sur le tibia (1) et d'une deuxième étoile de référence (5) disposée sur le fémur (2), peut déterminer les positions de l'axe du tibia et de l'axe du fémur l'un par rapport à l'autre pendant le test de mobilité, sachant que l'unité de calcul (10), lorsque le tibia (1) et le fémur (2) se trouvent dans une position pliée d'une première valeur d'angle prédéterminée, notamment de 30 degrés, par rapport à la position déployée, peut déterminer lors de l'exécution du test de mobilité les positions de l'axe du tibia et de l'axe du fémur l'un par rapport à l'autre pendant le test de mobilité en déterminant les positions et/ou orientations de la première et de la deuxième étoiles de référence (4, 5), sachant que l'unité de calcul (10), lorsque le tibia (1) et le fémur (2) se trouvent dans une position pliée d'une deuxième valeur d'angle prédéterminée, notamment de 90 degrés, par rapport à la position déployée, peut déterminer lors de l'exécution du test de mobilité les positions de l'axe du tibia et de l'axe du fémur pendant le test de mobilité en déterminant les positions et/ou orientations de la première et de la deuxième étoiles de référence (4, 5), et dans lequel l'unité de calcul (10) peut, à partir des positions déterminées des points axiaux, déterminer la stabilité et la mobilité du genou (3) ou de l'articulation du genou.

16. Dispositif selon la revendication précédente, dans lequel le dispositif comprend en outre un dispositif d'affichage, qui est relié au système de navigation (9) et/ou à l'unité de calcul (10) et sur lequel peuvent être représentés le tibia (1), le fémur (2), les points de référence (6, 7) et les points axiaux (15, 16).
